# EUROPEAN PATENT SPECIFICATION

(11) **EP 2 440 293 B1**
(45) Date of publication and mention of the grant of the patent: **19.04.2017**
(21) Application number: 10728339.2
(22) Date of filing: 08.06.2010
(51) Int. Cl.: A61B 17/00, A61B 90/00, A61B 5/055, G01R 33/28, G01R 33/48, A61N 7/02

(54) **MR IMAGING GUIDED ULTRASOUND THERAPY**
MRT-GEFÜHRTE ULTRASCHALLTHERPAIE
THÉRAPIE PAR ULTRASONS GUIDÉE PAR IMAGERIE PAR RÉSONANCE MAGNÉTIQUE

(30) Priority: 12.06.2009 EP 09162547
(43) Date of publication of application: 18.04.2012
(73) Proprietor: Koninklijke Philips N.V., 5656 AE Eindhoven (NL)
(72) Inventor: VIRTA, Tero Jouko Valtter, 5656 AE Eindhoven (NL); EHNHOLM, Gosta Jakob, 5656 AE Eindhoven (NL); IMMONEN, Paavo, 5656 AE Eindhoven (NL)
(74) Representative: Damen, Daniel Martijn
(86) International application number: PCT/IB2010/052537
(87) International publication number: WO 2010/143133

(56) References cited:
- WO-A1-2008/102293
- WO-A2-03/096883

## Description

### FIELD OF THE INVENTION

The description relates to the field of magnetic resonance (MR) imaging. It concerns a therapeutic system comprising an ultrasound therapy unit and a MR imaging unit. The invention is set out in the appended claims.

### BACKGROUND OF THE INVENTION

Ultrasound is becoming an increasingly desirable approach for specific therapeutic interventions. In particular, the use of high intensity focused ultrasound is currently being used as an approach for thermal therapeutic intervention for uterine fibroids and has been examined for possible uses in the treatment of liver, brain, prostate, and other cancerous lesions. Ultrasound has also been the subject of much research as a means for mediating clot dissolution (sonothrombolysis), and has been shown to increase the efficacy of existing medical treatments such as the use of tissue plasminogen activator (tPA) as a thrombolytic agent for stroke patients. Ultrasound mediated drug delivery and gene therapy is a further active area of research. Genetic expression of proteins in gene therapy and increased delivery of drugs in site-targeted therapies have potential to treat a wide variety of diseases with minimal side-effects. Another application for ultrasound therapy is non-invasive treatment for cosmetic means, e.g., removal of fat. The use of ultrasound in all of these applications is desirable because it allows the non-invasive treatment of deep tissues with little or no effect on overlying organs.

Ultrasound therapy for tissue ablation works by insonifying a tissue of interest with high intensity ultrasound that is absorbed and converted into heat, thereby raising the temperature of the respective tissues. As the temperature rises above 55 degree centigrade, coagulative necrosis of the tissues occurs resulting in immediate cell death. The transducers used in therapy can be outside the body or be inserted into the body e.g. through blood vessels, urethra, rectum etc. However, ultrasound therapy is not limited to tissue ablation, but also relates to the use of other types of ultrasound-based bio-effects, including hemostasis, drug or gene delivery, clot dissolution etc.

Nowadays, MR imaging guided high intensity focused ultrasound (MR HIFU) systems are commercially available. The first clinical application is the ablation of benign tumours in the uterus, so-called intrauterine fibroids. Therein a focused ultrasound beam is directed towards the abdomen. The ultrasound beam is used for heating a tumour through the skin and intervening tissue while MR imaging is used for monitoring the temperature distribution within the insonified region. The latter makes the procedure safe and efficient. MR imaging, in particular MR thermometry, is used for the non-invasive monitoring of such ablative thermal therapies. The reconstruction of thermographic MR images during ultrasound therapy is useful to provide feedback to ensure that adequate heating is accomplished at the intended location while safeguarding that other critical anatomic structures are left intact.

A therapeutic system comprising an ultrasound therapy unit and a MR imaging unit is generally known, e.g., from US 5,590,653. In the known system, the ultrasound applicator of the ultrasound therapy unit is integrated in the patient table that carries the patient to be treated. The MR imaging unit uses a RF coil for receiving the MR signals, which can be provided either on a surface film of a water bag used for coupling the high intensity ultrasound into the body of the patient, or on a intra cavity probe on which also the ultrasound applicator is provided.

A drawback of the known system is that the RF coil used for MR signal reception has to be properly arranged during each patient setup before treatment. A further drawback is that the RF coil comes into close contact with the coupling liquid (water, watery gel, oil) for coupling ultrasound into the body. This constitutes a safety risk. The RF coil is a loose part in the known system which is permanently moved for each patient setup. The RF coil and the cabling, via which the RF coil is connected to the receiver of the MR imaging unit, have to be carefully protected against water. WO2008/102293 discloses a therapeutic system as disclosed in the preamble of claim 1.

### SUMMARY OF THE INVENTION

From the foregoing it is readily appreciated that there is a need for an improved therapeutic system for MR imaging guided HIFU. It is consequently an object of the description to provide a therapeutic system which avoids the above-mentioned drawbacks and facilitates a good image quality close to the ultrasound applicator.

In accordance with the description a therapeutic system is disclosed. The system of the description comprises:
- an ultrasound therapy unit arranged to insonify at least a portion of a body of a patient with high intensity ultrasound, wherein the ultrasound therapy unit comprises an ultrasound applicator attached to a patient table carrying the body of the patient, and
- a MR imaging unit arranged to acquire MR signals from the portion of the body and to reconstruct a MR image from the MR signals, wherein the MR imaging unit comprises a RF receiving antenna for receiving the MR signals, the RF receiving antenna being integrally incorporated into the patient table.

The gist of the invention is the integration of both the ultrasound applicator and the RF receiving antenna into the patient table. The integrated RF receiving antenna is fixedly integrated into the the patient table. Therefore, the RF receiving antenna is not involved at all during patient setup. The usability of the system of the invention is thus significantly improved vis-a-vis the prior art. The antenna is automatically in the correct position relative to the ultrasound applicator, thereby enabling a good image quality in the region of insonification. Moreover, the RF receiving antenna is fully covered by the enclosures forming the patient table. In this way, the antenna can reliably be kept dry. The safety of the system according to the invention is improved because the RF receiving antenna (and the associated cabling) are electrically isolated from the patient by the enclosures of the patient table.

In accordance with a preferred embodiment of the description, the ultrasound applicator is attached to the patient table below a treatment hole formed in the patient table, wherein the high intensity ultrasound passes through the treatment hole during insonification. In this embodiment, the RF receiving antenna is preferably a RF coil surrounding the treatment hole. In this particularly practical arrangement, the ultrasound beam passes through the RF coil which results in a high image sensitivity in the region of the focus of the insonification. The system of the description preferably comprises a water bag containing a coupling fluid for coupling the high intensity ultrasound through the treatment hole into the body of the patient. The RF coil can be arranged within the enclosures of the patient table at the circumference of the treatment hole where it is reliably kept away from the coupling fluid.

According to a further preferred embodiment of the description, the RF receiving antenna comprises a watertight casing. In this way, a water contact of the electrically conductive parts of the antenna is prevented. Moreover, the RF receiving antenna may be located within a watertight compartment of the patient table, thereby providing a further protection against fluid contact. The watertight compartment may be arranged at the circumference of the treatment hole such that the RF coil located in the compartment surrounds the treatment hole.

According to yet a further preferred embodiment of the description, the RF receiving antenna is connected to a receiver of the MR imaging unit via a RF cable which is also integrally incorporated into the patient table. This means that both the RF antenna and the associated cabling are integrated into the patient table. In this embodiment a particularly high safety level is achieved. A reliable protection against RF heating is obtained because a direct contact between the cabling and the body of the patient is prevented by the enclosures of the patient table. Moreover, a filter element may be incorporated into the patient table, which filter element is arranged around the RF cable for blocking the induction of currents within the RF cable. Such a RF cable trap blocks stray RF currents from flowing on the shield conductors of the RF cable. The filter couples with the RF cable to present a high signal attenuating impedance at the resonance frequency of the MR imaging unit. In accordance with the description, the RF cable and the filter can be integrated into the patient table. The present invention is set out in the appended claims. The embodiments, examples or aspects according to the present description that do not fall within the scope of said claims are provided for illustrative purposes only and do not form part of the invention.

### BRIEF DESCRIPTION OF THE DRAWINGS

The enclosed drawings disclose preferred embodiments. It should be understood, however, that the drawings are designed for the purpose of illustration only and not as a definition of the limits of the description. In the drawings
Fig. 1 schematically shows a therapeutic system of the description,
Fig. 2 shows a schematical cut side-view of the system of the description, and
Fig. 3 shows a more detailed lateral cut of the patient table shown in Figure 2.

### DETAILED DESCRIPTION OF EMBODIMENTS

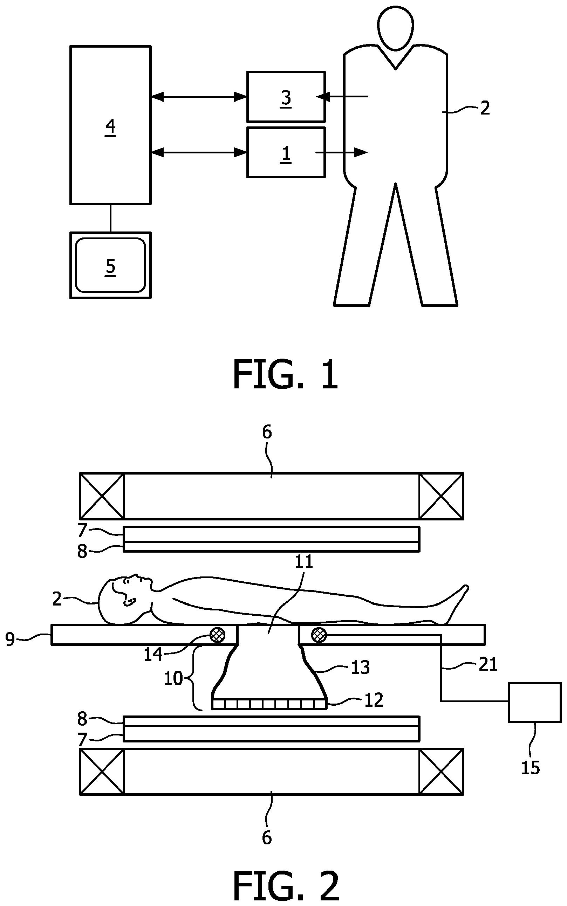

With reference to Figure 1, the therapeutic system according to an embodiment of the description comprises an ultrasound therapy unit 1 which is arranged to insonify at least a portion of a body 2 of a patient with a high intensity focused ultrasound beam. The system further comprises a MR imaging unit 3 that acquires MR signals from the portion of the body 2 and reconstructs thermographic MR images from the acquired MR signals, e.g. on the basis of the local proton resonance frequency shift (PRFS). The ultrasound therapy unit 1 and the MR imaging unit 3 are connected to a common control unit 4 of the system. The control unit 4 controls the position, direction and/or focusing of the high intensity ultrasound beam. The control unit 4 processes data obtained via the MR imaging unit 3, for example during the treatment planning phase. Moreover, the control unit 4, which might be a micro computer as it is commonly used in medical imaging and therapeutic devices, computes a thermographic MR image, i.e. the spatial temperature distribution in the treated portion of the body 2 on the basis of the MR signals acquired via the MR imaging unit 3. The system is able to perform real-time MR thermography and - based on the measured and reconstructed temperature distribution - control the therapeutic action, i.e. causing the targeted elevation of local temperature in the tissue by means of suitably adjusting the focused ultrasound beam of the ultrasound therapy unit 1. The thermographic MR image is presented via a display unit 5 to an operator of the system.

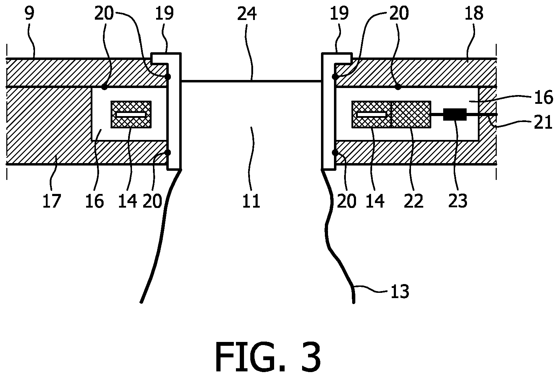

With continuing reference to Figure 1 and with further reference to Figures 2 and 3, the system comprises superconducting or resistive main magnet coils 6 such that a substantially uniform, temporally constant main magnetic field is created along a z-axis through an examination volume. A magnetic resonance generation and manipulation system applies a series of RF pulses and switched magnetic field gradients to invert or excite nuclear magnetic spins, induce magnetic resonance, refocus magnetic resonance, manipulate magnetic resonance, spatially and otherwise encode the magnetic resonance, saturate spins, and the like to perform MR imaging. More specifically, a gradient pulse amplifier (not shown) applies current pulses to whole-body gradient coils 7 along x, y and z-axes of the examination volume. A digital RF frequency transmitter (not shown) transmits RF pulses or pulse packets to a whole-body volume RF coil 8 to transmit RF pulses into the examination volume. A typical MR imaging sequence is composed of a packet of RF pulse segments of short duration which taken together with each other and any applied magnetic field gradients achieve a selected manipulation of nuclear magnetic resonance. The RF pulses are used to saturate, excite resonance, invert magnetization, refocus resonance, or manipulate resonance and select a portion of a body 2 positioned on a patient table 9 in the examination volume.

An ultrasound applicator 10 of the ultrasound therapy unit 1 is attached below a treatment hole 11 formed in the patient table 9. The ultrasound applicator 10 comprises an ultrasound transducer 12 for generating high intensity ultrasound. Provision is made for a bag 13 containing a coupling fluid (e.g. water, watery gel, oil) for coupling the intense ultrasound into the body 2 through the treatment hole 11. The treatment hole 11 is of round shape and located essentially in the middle of the patient table 9. The treatment hole 11 is covered by an ultrasound membrane 24, such as a Mylar plastic film diaphragm.

For generation of MR images of the limited region of the body 2 to which ultrasound is applied, a RF coil 14 is integrated into the patient table 9 contiguous to the region selected for imaging. The RF coil 14 is used to receive MR signals induced by body-coil 8 RF transmissions. The resultant MR signals are picked up by the RF coil 14 and demodulated by a receiver 15 preferably including a preamplifier (not shown).

The control unit 4 controls the system to generate any of a plurality of MR imaging sequences, such as echo planar imaging (EPI), echo volume imaging, gradient and spin echo imaging, fast spin echo imaging, and the like. For the selected sequence, the receiver 15 receives a single or a plurality of MR data lines in rapid succession following each RF excitation pulse. A data acquisition system (not shown) performs analog-to-digital conversion of the received signals and converts each MR data line to a digital format suitable for further processing. Ultimately, the digital raw image data is reconstructed into an image representation. The MR image may represent a planar slice through the patient, an array of parallel planar slices, a three-dimensional volume, or the like. The image is then stored in an image memory where it may be accessed for converting slices, projections, or other portions of the image representation into appropriate format for visualization via the display unit 5.

As shown in detail in Figure 3, the RF coil 14 is located within a watertight compartment 16 which is arranged at the circumference of the treatment hole 11. In this way, the RF coil 14 forms a loop that surrounds the treatment hole 11. The compartment 16, is formed by a base part 17 and a top part 18 of the enclosure of the patient table 9 as well as by a ring-shaped insert 19 defining the treatment hole 11. The RF coil 14 is in this way completely hidden under the enclosure parts of the patient table. Provision is made for sealings 20 in order to achieve watertightness. The RF coil 14 is made from vacuum formed plates that are glued together such that the electrically conductive parts of the RF coil 14 are enclosed within a further watertight casing. The RF coil 14 may equally well be manufactured by injection molding. A high level of safety is achieved in this way even though the RF coil 14 is located in direct vicinity of the ultrasound coupling fluid, which is in the bag 13 and/or in direct contact with the body 2 of the patient. The RF coil 14 itself is watertight and the RF coil 14 is moreover arranged in a fully watertight compartment 16 of the patient table 9. Also a RF cable 21, via which a tuning and matching module 22 of the RF coil 14 is connected to the receiver 15, is integrated into the patient table 9. In this way, the enclosures 17, 18 of the patient table provide an electrical (and thermal) insulation between the cable 21 and the body 2 of the patient. A filter element 23, which is arranged around the RF cable 21 for blocking the induction of currents, is incorporated into the patient table 9 as well.

## Claims

1. Therapeutic system comprising:
- a patient table (9) configured to carry a patient's body (2)
- an ultrasound therapy unit (1) arranged to insonify at least a portion of the body (2) of the patient with high intensity ultrasound, wherein the ultrasound therapy unit (1) comprises an ultrasound applicator (10) attached to the patient table (9) carrying the body (2) of the patient, and
- a MR imaging unit (3) arranged to acquire MR signals from the portion of the body (2) and to reconstruct a MR image from the MR signals, wherein the MR imaging unit (3) comprises a RF receiving antenna (14) for receiving the MR signals, and **characterized in that** the RF receiving antenna (14) is integrally incorporated into the patient table (9), so as to be fully covered by the enclosures forming the patient table.

2. Therapeutic system according to claim 1, wherein the ultrasound applicator (10) is attached to the patient table (9) below a treatment hole (11) formed in the patient table, the high intensity ultrasound passing through the treatment hole (11) during insonification.

3. Therapeutic system according to claim 2, wherein the RF receiving antenna (14) is a RF coil surrounding the treatment hole (11).

4. Therapeutic system according to any one of claims 1-3, further comprising a water bag (13) containing a coupling fluid for coupling the high intensity ultrasound through the treatment hole (11) into the body (2) of the patient.

5. Therapeutic system according to any one of claims 1-4, wherein the RF receiving antenna (14) comprises a watertight casing.

6. Therapeutic system according to any one of claims 1-5, wherein the RF receiving antenna (14) is located within a watertight compartment (16) of the patient table (9).

7. Therapeutic system according to claims 4 and 6, wherein the watertight compartment (16) is arranged at the circumference of the treatment hole (11).

8. Therapeutic system according to any one of claims 1-7, wherein the RF receiving antenna (14) is connected to a receiver (15) of the MR imaging unit (3) via a RF cable (21) which is integrally incorporated into the patient table (9).

9. Therapeutic system according to claim 8, wherein a filter element (23) is incorporated into the patient table (9), which filter element is arranged around the RF cable for blocking the induction of currents within the RF cable (21).

## Patentansprüche

1. Therapeutisches System, das Folgendes umfasst:
- einen Patiententisch (9), der konfiguriert ist, um einen Patientenkörper (2) zu tragen,
- eine Ultraschalltherapieeinheit (1), die dafür ausgelegt ist, mindestens einen Teil des Körpers (2) des Patienten mit hochintensivem Ultraschall zu beschallen, wobei die Ultraschalltherapieeinheit (1) einen Ultraschallapplikator (10) umfasst, der an dem Patiententisch (9) angebracht ist, welcher den Körper (2) des Patienten trägt, und
- eine MR-Bildgebungseinheit (3), die dafür ausgelegt ist, MR-Signale aus dem Teil des Körpers (2) zu erfassen und anhand der MR-Signale ein MR-Bild zu rekonstruieren, wobei die MR-Bildgebungseinheit (3) eine HF-Empfangsantenne (14) zum Empfangen von MR-Signalen umfasst, und **dadurch gekennzeichnet, dass** die HF-Empfangsantenne (14) integral in den Patiententisch (9) aufgenommen ist, sodass sie durch die den Patiententisch bildenden Einfassungen vollständig abgedeckt ist.

2. Therapeutisches System nach Anspruch 1, wobei der Ultraschallapplikator (10) unterhalb einer in den Patiententisch eingeformten Behandlungsöffnung (11) an dem Patiententisch (9) angebracht ist, wobei der hochintensive Ultraschall während der Beschallung durch die Behandlungsöffnung (11) hindurchtritt.

3. Therapeutisches System nach Anspruch 2, wobei die HF-Empfangsantenne (14) eine die Behandlungsöffnung (11) umgebende HF-Spule ist.

4. Therapeutisches System nach einem der Ansprüche 1 bis 3, weiterhin umfassend einen Wasserbeutel (13) enthaltend ein Kopplungsfluid zum Einkoppeln des hochintensiven Ultraschalls durch die Behandlungsöffiiung (11) in den Körper (2) des Patienten.

5. Therapeutisches System nach einem der Ansprüche 1 bis 4, wobei die HF-Empfangsantenne (14) ein wasserdichtes Gehäuse umfasst.

6. Therapeutisches System nach einem der Ansprüche 1 bis 5, wobei die HF-Empfangsantenne (14) in einem wasserdichten Abteil (16) des Patiententischs (9) angeordnet ist.

7. Therapeutisches System nach den Ansprüchen 4 und 6, wobei das wasserdichte Abteil (16) am Umfang der Behandlungsöffnung (11) angeordnet ist.

8. Therapeutisches System nach einem der Ansprüche 1 bis 7, wobei die HF-Empfangsantenne (14) über ein HF-Kabel (21), das integral in den Patiententisch (9) aufgenommen ist, mit einem Empfänger (15) der MR-Bildgebungseinheit (3) verbunden ist.

9. Therapeutisches System nach Anspruch 8, wobei ein Filterelement (23) in den Patiententisch (9) aufgenommen ist, wobei das Filterelement um das HF-Kabel herum angeordnet ist, um die Induktion von Strömen im HF-Kabel (21) zu blockieren.

## Revendications

1. Système thérapeutique comprenant :
- une table de patient (9) configurée pour porter un corps de patient (2)
- une unité de thérapie par ultrasons (1) conçue pour insonifier au moins une partie du corps (2) du patient à l'aide d'ultrasons de haute intensité, dans lequel l'unité de thérapie par ultrasons (1) comprend un applicateur d'ultrasons (10) fixé à la table de patient (9) portant le corps (2) du patient, et
- une unité d'imagerie par RM (3) conçue pour acquérir les signaux RM de la partie du corps (2) et pour reconstruire une image par RM à partir des signaux RM, dans lequel l'unité d'imagerie par RM (3) comprend une antenne de réception RF (14) destinée à recevoir les signaux RM, et **caractérisé en ce que** l'antenne de réception RF (14) est encastrée de manière solidaire dans la table de patient (9), de sorte à être totalement couverte par les enveloppes formant la table de patient.

2. Système thérapeutique selon la revendication 1, dans lequel l'applicateur d'ultrasons (10) est fixé à la table de patient (9) sous un orifice de traitement (11) formé dans la table de patient, les ultrasons de haute intensité passant à travers l'orifice de traitement (11) au cours de l'insonification.

3. Système thérapeutique selon la revendication 2, dans lequel l'antenne de réception RF (14) est une bobine à RF entourant l'orifice de traitement (11).

4. Système thérapeutique selon l'une quelconque des revendications 1 à 3, comprenant en outre une poche d'eau (13) contenant un fluide de couplage pour coupler les ultrasons de haute intensité à travers le trou de traitement (11) dans le corps (2) du patient.

5. Système thérapeutique selon l'une quelconque des revendications 1 à 4, dans lequel l'antenne de réception RF (14) comprend un carter étanche à l'eau.

6. Système thérapeutique selon l'une quelconque des revendications 1 à 5, dans lequel l'antenne de réception RF (14) est située à l'intérieur d'un compartiment étanche à l'eau (16) de la table de patient (9).

7. Système thérapeutique selon les revendications 4 et 6, dans lequel le compartiment étanche à l'eau (16) est placé sur la circonférence de l'orifice de traitement (11).

8. Système thérapeutique selon l'une quelconque des revendications 1 à 7, dans lequel l'antenne de réception RF (14) est branchée à un récepteur (15) de l'unité d'imagerie par RM (3) via un câble RF (21) qui est encastré de manière solidaire dans la table de patient (9).

9. Système thérapeutique selon la revendication 8, dans lequel un élément filtrant (23) est encastré dans la table de patient (9), lequel élément filtrant est placé autour du câble RF pour bloquer l'induction de courants dans le câble RF (21).
